(19) Europäisches Patentamt
European Patent Office
Office européen des brevets

(11) EP 2 233 154 A1

(12) **EUROPEAN PATENT APPLICATION**
published in accordance with Art. 153(4) EPC

(43) Date of publication:
**29.09.2010 Bulletin 2010/39**

(21) Application number: **08856959.5**

(22) Date of filing: **05.12.2008**

(51) Int Cl.:
***A61K 47/44*** *(2006.01)* ***A61K 8/04*** *(2006.01)*
***A61K 8/92*** *(2006.01)* ***A61K 31/07*** *(2006.01)*
***A61K 31/355*** *(2006.01)* ***A61K 31/375*** *(2006.01)*
***A61K 45/00*** *(2006.01)* ***A61K 47/34*** *(2006.01)*
***A61P 17/00*** *(2006.01)* ***A61Q 19/00*** *(2006.01)*

(86) International application number:
**PCT/JP2008/072201**

(87) International publication number:
**WO 2009/072629 (11.06.2009 Gazette 2009/24)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **07.12.2007 JP 2007317631**
**04.12.2008 JP 2008309771**

(71) Applicant: **Shiseido Company, Ltd.**
**Chuo-ku**
**Tokyo 104-8010 (JP)**

(72) Inventors:
- **TAKEOKA, Eriko**
**Yokohama-shi**
**Kanagawa 224-8558 (JP)**
- **TESHIGAWARA, Takashi**
**Yokohama-shi**
**Kanagawa 224-8558 (JP)**
- **KUSABA, Kentaro**
**Yokohama-shi**
**Kanagawa 224-8558 (JP)**
- **MATSUO, Akira**
**Yokohama-shi**
**Kanagawa 224-8558 (JP)**
- **TAMURA, Junko**
**Yokohama-shi**
**Kanagawa 224-8558 (JP)**

(74) Representative: **Gendron, Vincent Christian et al**
**S.A. Fedit-Loriot**
**38, avenue Hoche**
**75008 Paris (FR)**

# (54) SKIN EXTERNAL PREPARATION

(57) Object

To provide skin external preparations **characterized in that** the initial permeation rate of oil-soluble drugs (e.g. retinol and tocopherol acetate) is controlled to achieve their sustained release to thereby reduce skin irritation and that they also possess a fresh feel while featuring high degrees of safety and stability.

Means of attaining the object

A skin external preparation which comprises a finely dispersed, oil-soluble drug containing wax composition, the composition containing a solid or semisolid wax, a nonionic surfactant, an aqueous dispersion medium, and an oil-soluble drug, the mass ratio of the nonionic surfactant to the wax being 1.0 or more, and the wax, with the oil-soluble drug contained therein, being finely dispersed in solid or semisolid form in the aqueous dispersion medium.

[FIG. 1]

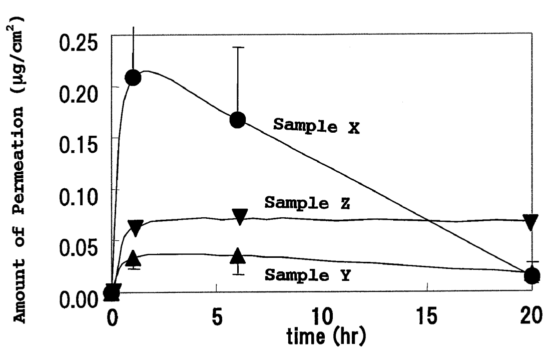


EP 2 233 154 A1

## Description

TECHNICAL FIELD

The present invention relates to skin external preparations that comprise a finely dispersed, oil-soluble drug containing wax composition which is **characterized in that** wax incorporating an oil-soluble drug is finely dispersed in solid or semisolid form in an aqueous dispersion medium. The present invention particularly relates to skin external preparations that allow the oil-soluble drug to be released at a controlled rate so that the sustained-release effect is sufficiently enhanced to suppress skin irritation and which are safe, feature good use properties (high degree of freshness) and are highly stable.

BACKGROUND ART

Conventionally, in cases where oil-soluble drugs such as retinol (vitamin A) and its derivatives need be incorporated in skin external preparations, cosmetics and the like, forms of the preparations or cosmetics have been limited to ones of high oil content that are highly viscous and which are liquid at ordinary temperatures. See, for example, Patent Document 1 which describes an oily composition **characterized in that** a solid phase containing a water-soluble or water-dispersible effective substance is dispersed as fine particles in an oil phase; this reference notes that an oil-soluble effective substance (e.g., tocopherol or vitamin A) may be contained in the oil phase. However, the oily composition of Patent Document 1 has the oil phase (comprising an emulsifying agent and an oily ingredient) incorporated in high proportion and suffers a disadvantage that it fails to provide a fresh feeling during use. If the content of the oil phase in the system is lowered, an improvement is realized to provide a fresh feeling during use, but on the other hand, the relative amount of the oil-soluble drug that is distributed to the skin is increased to cause the problem of skin irritation. To state more specifically, the oil-soluble drugs such as retinol (vitamin A) and its derivatives have an irritating effect on the skin and in systems of low oil content, it is difficult to control their distribution to the oil phase and they tend to permeate into the skin in an excessive amount, producing a concern about increased irritation of the skin, which is not desirable from a safety viewpoint.

To eliminate greasiness, glare and other disadvantages that result from the incorporation of wax in cosmetics, the applicant of the subject application has proposed a technology in which wax is finely dispersed in a solvent to make a wax emulsion, thereby producing a finely dispersed wax composition that is not only free from the above-mentioned defects but also characterized by high degrees of stability and safety (see, for example, Patent Documents 2 to 4). However, each of the finely dispersed wax compositions described in Patent Documents 2 to 4 is intended to utilize the performance such as lustering and shape-retaining properties of wax itself so that they can be applied to hair cosmetics such as setting lotions and hair mousses and lustering preparations, and none of these patent documents describe or suggest the idea of the present invention, i.e., controlling the release rate of oil-soluble drugs to enhance their sustained-release property and thereby suppress irritation on the skin.

Patent Documents 5 and 6 also describe hair cosmetic compositions comprising microdispersions of wax; however, the technology disclosed in each of these references is also intended to utilize the performance such as lustering and shape-retaining properties of wax itself so that they can be applied to hair cosmetics such as setting lotions and hair mousses and lustering preparations, and none of these patent documents describe or suggest the idea of the present invention, i.e., controlling the release rate of oil-soluble drugs to enhance their sustained-release property and thereby suppress irritation on the skin.

Patent Document 1: JP 11-113487 A (e.g., paragraph number [0012])
Patent Document 2: JP 10-324617 A
Patent Document 3: JP 11-286418 A
Patent Document 4: JP 11-263915 A
Patent Document 5: JP 4-230616 A
Patent Document 6: JP 3-2112 A

DISCLOSURE OF THE INVENTION

PROBLEMS TO BE SOLVED BY THE INVENTION

The present invention has been accomplished in light of the above-mentioned problems of the prior art and its object is to provide skin external preparations that are **characterized in that** the initial permeation rate of oil-soluble drugs is controlled to achieve their sustained release to thereby reduce skin irritation and which also possess a fresh

feel while featuring high degrees of safety and stability.

MEANS FOR SOLVING THE PROBLEMS

To solve the aforementioned problems, the present inventors made intensive studies and found that by containing an oil-soluble drug within fine wax particles and dispersing them in an aqueous dispersion medium, the oil-soluble drug is "covered" with the wax, with the result that the rate at which the oil-soluble drug of this structure, i.e., being contained in the wax, permeates to the outside of the wax is controlled to achieve sustained release of the oil-soluble drug component, thereby producing compositions that would cause no irritation on the skin and which were safe while possessing a fresh feel; the present invention has been accomplished on the basis of this finding.

Thus, the present invention provides a skin external preparation which comprises a finely dispersed, oil-soluble drug containing wax composition, the composition containing a solid or semisolid wax, a nonionic surfactant, an aqueous dispersion medium, and an oil-soluble drug, the mass ratio of the nonionic surfactant to the wax being 1.0 or more, and the wax, with the oil-soluble drug contained therein, being finely dispersed in solid or semisolid form in the aqueous dispersion medium.

In a preferred embodiment, the oil-soluble drug is one or more substances selected from among vitamin A and its derivatives, vitamin $B_2$ derivatives, vitamin $B_6$ derivatives, vitamin D and its derivatives, vitamin E and its derivatives, essential fatty acids, ubiquinone and its derivatives, K vitamins, resorcin derivatives, glycyrrhetinic acid and its derivatives, oil-soluble vitamin C derivatives, steroid compounds, benzyl nicotinate, trichlorocarbanilide, trichlorohydroxydiphenylether, stearyl glycyrrhetinate, γ-orizanol, and dibutylhydroxytoluene.

In another preferred embodiment, the weight-averaged HLB for all nonionic surfactants in the finely dispersed, oil-soluble drug containing wax composition is in the range of 10-15.

In yet another preferred embodiment, one or more substances selected from among polyoxyethylene alkyl ethers, polyoxyethylene-polyoxypropylene alkyl ethers, polyoxyethylene glyceryl ether fatty acid esters, and polyoxyethylene castor oil or polyoxyethylene hydrogenated castor oil and their derivatives are contained as the nonionic surfactant.

In still another preferred embodiment, polyoxyethylene alkyl ethers and/or polyoxyethylene-polyoxypropylene alkyl ethers and polyoxyethylene glyceryl ether fatty acid esters are contained as nonionic surfactants.

In another preferred embodiment, the polyoxyethylene alkyl ethers and polyoxyethylene-polyoxypropylene alkyl ethers are one or more substances selected from among compounds represented by the following formulas (I) and/or (II):

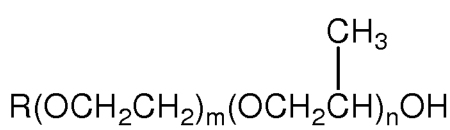

(I)

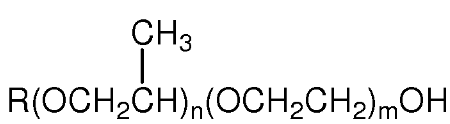

(II)

[in the formulas (I) and (II), R represents an alkyl or alkenyl group having 12-24 carbon atoms, m represents a number of 5-30, and n represents a number of 0-5].

In yet another preferred embodiment, the finely dispersed, oil-soluble drug containing wax composition is obtained by such a process that a system containing a wax that is solid or semisolid at ordinary temperatures, a nonionic surfactant, an aqueous dispersion medium, and an oil-soluble drug, with the mass ratio of the nonionic surfactant to the wax being 1.0 or more, is heated to a temperature not lower than the melting point of the wax but within the solubilizing temperature range so that the wax becomes solubilized and the system is thereafter cooled to ordinary temperatures.

EFFECTS OF THE INVENTION

According to the present invention, there are provided skin external preparations that allow oil-soluble drugs

to be released at a controlled rate so that the sustained-release effect is sufficiently enhanced to suppress skin irritation and which are safe, feature good use properties (high degree of freshness) and are highly stable.

BEST MODES FOR CARRYING OUT THE INVENTION

Hereinafter, the present invention is described in detail.

In the following description, "POE" means polyoxyethylene and "POP" polyoxypropylene.

[Finely dispersed, oil-soluble drug containing wax composition]

The finely dispersed, oil-soluble drug containing wax composition which is used in the skin external preparations of the present invention contains a wax that is solid or semisolid at ordinary temperatures, a nonionic surfactant, an aqueous dispersion medium, and an oil-soluble drug, and the wax, with the oil-soluble drug contained therein, being finely dispersed in solid or semisolid form in the aqueous dispersion medium.

<Wax>

The wax which is used in the present invention assumes a solid or semisolid state at ordinary temperatures and specific examples include, but are not limited to, beeswax, candelilla wax, cotton wax, carnauba wax, bayberry wax, insect wax (wax secreted by *Ericerus pela*), spermaceti, montan wax, bran wax, lanolin, capok wax, Japan wax, lanolin acetate, liquid lanolin, sugar cane wax, esters of lanolin fatty acids and isopropyl alcohol, hexyl laurate, reduced lanolin, jojoba wax, hard lanolin, shellac wax, beeswax, microcrystalline wax, paraffin wax, POE lanolin alcohol ethers, POE lanolin alcohol acetates, POE cholesterol ethers, esters of lanolin fatty acids and polyethylene glycol, fatty acid glycerides, hydrogenated castor oil, petrolatum, and POE hydrogenated lanolin alcohol ethers.

It should be noted that the above-listed waxes may be used in admixture and even if they are mixed with other solid or liquid oil components, the mixtures can be used within the range where they are in solid or semisolid form at ordinary temperatures.

Such oil components may include, but are not limited to, the following.

Liquid oils and fats include avocado oil, camellia oil, turtle oil, macadamia nut oil, corn oil, mink oil, olive oil, rapeseed oil, yolk oil, sesame oil, persic oil, wheat germ oil, sasanqua oil, castor oil, linseed oil, safflower oil, cottonseed oil, Perilla oil, soybean oil, peanut oil, tea seed oil, *Torreya nucifera* oil, rice-bran oil, Chinese tung oil, Japanese tung oil, jojoba oil, germ oil, triglycerin, glyceryl trioctanoate, pentaerythritol tetraoctanoate, and glyceryl triisopalmitate.

Solid oils and fats include cacao oil, coconut oil, hydrogenated coconut oil, palm oil, palm kernel oil, Japan wax kernel oil, and hardened oils. Hydrocarbon oils include liquid paraffin, ozokerite, squalene, pristane, paraffin, and squalane.

The amount of the wax to be used in the present invention preferably ranges from 0.01 to 25 mass%, more preferably from 0.1 to 15 mass%, in the total amount of the finely dispersed, oil-soluble drug containing wax composition. If the wax content is less than 0.01 mass%, it is difficult to contain an adequate amount of the oil-soluble drug in the wax. On the other hand, if the wax is used in an amount greater than 25 mass%, formulations are difficult to prepare.

From the viewpoint of imparting a fresh feeling during use, the oil component is preferably contained in an amount of about 0.1 to 5 mass% in the finely dispersed, oil-soluble drug containing wax composition.

<Nonionic surfactant>

The nonionic surfactant is not particularly limited as long as it is of types that can generally be used in cosmetics, but in the present invention, it is preferred that the weight-averaged HLB for all the nonionic surfactants in the finely dispersed, oil-soluble drug containing wax composition is in the range of 10-15, more preferably 11-14, and even more preferably 12-13. By using nonionic surfactants providing HLB values within the stated ranges, one can obtain such a clear composition that the wax has been solubilized in a hot state (e.g., within the solubilizing temperature range not lower than the melting point of the wax). HLB can be calculated by the Kawakami Equation represented by the following formula 1:

[Formula 1]

$$HLB = 7 + 11.7 \cdot \log(MW/MO)$$

(where MW represents the molecular weight of the hydrophilic moiety and MO represents the molecular weight of the lipophilic moiety).

In the present invention, the nonionic surfactant that may be used with particular preference is one or more substances selected from among POE alkyl ethers, POE-POP alkyl ethers, POE glyceryl ether fatty acid esters, and POE castor oil or POE hydrogenated castor oil and their derivatives. Among these substances, POE alkyl ethers and POE-POP alkyl ethers are particularly advantageous to use because the prepared fine dispersion of the oil-soluble drug containing wax has such good stability with time that the fine particles of the wax will not agglomerate or otherwise deteriorate over time to cause a change in appearance (lowered transparency) or creaming of the dispersed particles.

As will be described later in this specification, the finely dispersed, oil-soluble drug containing wax composition which is used in the present invention is advantageously produced by a process comprising the steps of heating the system until the wax is solubilized and then cooling the system to ordinary temperatures so that the wax becomes finely dispersed. If one or more substances selected from between POE alkyl ethers and POE-POP alkyl ethers are used in combination with POE glyceryl ether fatty acid esters as nonionic surfactants, the rate of wax solubilization can be remarkably increased to achieve an improvement in production efficiency.

As the above-mentioned POE alkyl ethers and POE-POP alkyl ethers, one or more substances selected from among compounds represented by the following formulas (I) and/or (II) are preferably used:

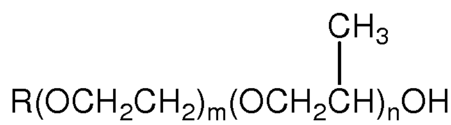

(I)

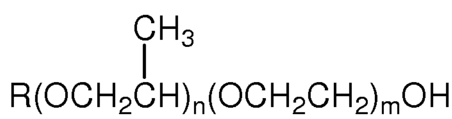

(II)

[in the formulas (I) and (II), R represents an alkyl or alkenyl group having 12-24 carbon atoms, m represents a number of 5-30, and n represents a number of 0-5].

Examples of the above-described POE alkyl ethers and POE-POP alkyl ethers include POE lauryl ethers, POE cetyl ethers, POE stearyl ethers, POE oleyl ethers, POE behenyl ethers, POE decyltetradecyl ethers, POE monobutyl ethers, POE 2-decyltetradecyl ethers, POE hydrogenated lanolin, POE glyceryl ethers, POE-POP lauryl ethers, POE-POP cetyl ethers, POE-POP stearyl ethers, POE-POP oleyl ethers, POE-POP behenyl ethers, POE-POP decyltetradecyl ethers, POE-POP monobutyl ethers, POE-POP 2-decyltetradecyl ethers, POE-POP hydrogenated lanolin, and POE-POP glyceryl ethers.

Examples of the above-mentioned POE glyceryl ether fatty acid esters include POE glyceryl ether monostearic acid ester, POE glyceryl ether monoisostearic acid ester, and POE glyceryl ether triisostearic acid ester.

Examples of the above-mentioned POE castor oil or POE hydrogenated castor oil and their derivatives include POE castor oil, POE hydrogenated castor oil, POE hydrogenated castor oil monoisostearate, POE hydrogenated castor oil triisostearate, POE hydrogenated castor oil monopyroglutamic acid monoisostearic acid diester, and POE hydrogenated castor oil maleate.

In the present invention, any nonionic surfactants other than those listed above can also be used, and examples include: POE sorbitan fatty acid esters, such as POE sorbitan monooleate and POE sorbitan tetraoleate; POE sorbitol fatty acid esters, such as POE sorbitol monolaurate, POE sorbitol monooleate, POE sorbitol pentaoleate, and POE sorbitol monostearate; POE fatty acid esters, such as POE monooleate and ethylene glycol distearate; POE alkylphenyl ethers, such as POE octylpyhenyl ether, POE nonylphenyl ether, and POE dinonylphenyl ether; pluronics such as pluronic; tetraPOE-tetraPOP ethylenediamine condensates such as Tetronic; POE beeswax-lanolin derivatives such as POE sorbitol beeswax; alkanol amides, such as coconut oil fatty acid diethanol amides, lauric acid monoethanol amide, and fatty acid isopropanol amides; as well as POE propylene glycol fatty acid esters, POE alkylamines, POE fatty acid amides, sucrose fatty acid esters, POE nonylphenyl formaldehyde condensate, alkylethoxydimethyl amine oxides, and trioleyl phosphate.

The proportion of the nonionic surfactant relative to the wax for use in the present invention is 1.0 or more (in

mass ratio) in the total amount of the finely dispersed, oil-soluble drug containing wax composition, with 1.1 or more being preferred. If this mass ratio is less than 1.0, a composition of high stability is difficult to obtain. The upper limit of the above-defined mass ratio is not particularly limited but it is preferably not greater than about 5.0, more preferably not greater than about 3.0. If the value of this mass ratio is unduly great, the feeling on use tends to deteriorate.

<Aqueous dispersion medium>

In the present invention, the wax described above is finely dispersed as fine particles in an aqueous dispersion medium. Water may be used alone as the aqueous dispersion medium; alternatively, it may be used as liquid mixtures of water and other substances such as ethanol, glycerin, polyethylene glycol, propylene glycol, dipropylene glycol, 1,3-butanediol, xylitol, sorbitol, maltitol, chondroitin sulfate, mucoitin sulfate, calonic acid, atherocollagen, cholesteryl-12-hydroxystearate, sodium lactate, bile acid salts, short-chain soluble collagens, diglycerin(EO)PO adducts, chestnut rose extract, yarrow extract, and melilot extract. Note that the fine particles of wax (finely divided wax) are preferably dispersed in aqueous solvents as finely divided wax having a particle size of no more than 500 nm.

<Oil-soluble drug>

In the present invention, the oil-soluble drug is contained in the finely divided wax so that it undergoes sustained release for absorption into the skin, and examples include oil-soluble drug components, such as skin lightening agents, humectants, antiinflammatories, antimicrobials, hormone preparations, vitamins, various amino acids and their derivatives, as well as enzymes, antioxidants, hair growth tonics, and UV absorbers. Specific examples include but are not limited to: vitamin A (retinol) and its derivatives (e.g., retinol acetate and retinol palmitate), vitamin $B_2$ derivatives (e.g., riboflavin acetate), vitamin $B_6$ derivatives (e.g., pyridoxine dicaprylate, pyridoxine dipalmitate, and pyridoxine dilaurylate), vitamin D (= calcipherol) and its derivatives (e.g., ergocalcipherol and cholecalcipherol), vitamin E (= tocopherol) and its derivatives [e.g., vitamin E acetate (= tocopherol acetate)], essential fatty acids [e.g., linoleic acid, linolenic acid, arachidonic acid, eicosapentaenoic acid, docosahexaenoic acid, and plant-derived extracts containing these acids (e.g., *Biota orientalis*)], ubiquinone (= coenzyme Q10) and its derivatives, K vitamins (e.g., phylloquinone, menaquinone, and menadione), resorcine derivatives (e.g., 4-alkylresorcinol derivatives and/or their salts), glycyrrhetic acid and its derivatives (e.g., stearyl glycyrrhetinate), oil-soluble vitamin C derivatives [e.g., vitamin C dipalmitate (= ascorbyl dipalmitate) and ascorbyl stearate], steroid compounds (e.g., estrogen and androgen), benzyl nicotinate (an ingredient in hair growth tonics), trichlorocarbanilide (an ingredient in bactericides), trichlorohydroxydiphenyl ether (an ingredient in antiseptics), γ-orizanol (an ingredient in antioxidants), dibutylhydroxytoluene (an ingredient in antioxidants), and UV absorbers such as octyl methoxycinnamate, 4-(1,1-dimethylethyl)-4'-methoxybenzoylmethane, and octocrylene. The oil-soluble drugs may be used either alone or in combination.

The amount of the oil-soluble drug to be used in the present invention preferably ranges from 0.0001 to 10 mass%, more preferably from 0.001 to 5 mass%, in the total amount of the finely dispersed, oil-soluble drug containing wax composition. If the amount of the oil-soluble drug is less than 0.0001 mass%, its efficacy is not fully exhibited; on the other hand, if the oil-soluble drug is used in an amount exceeding 10 mass%, there is no expecting a further improvement in efficacy that is commensurate with the increased amount of its use.

<Process for production>

In the finely dispersed, oil-soluble drug containing wax composition that is to be used in the present invention, the wax, with the oil-soluble drug contained in it, is finely dispersed in solid or semisolid form in the aqueous dispersion medium. The process for producing such wax composition is not particularly limited but the following process may be given as an advantageous example.

In the process, a system containing the above-described wax, nonionic surfactant, aqueous dispersion medium, and oil-soluble drug is heated to a temperature not lower than the melting point of the wax but within the solubilizing temperature range so that the system is rendered to a solubilized state and is thereafter cooled to ordinary temperatures. A more specific advantageous embodiment may be exemplified by the following: the nonionic surfactant and optionally a polyhydric alcohol are dissolved in ion-exchange water and to the solution being heated to 85-95˚C, the wax (e.g., carnauba wax, etc.) is added and the resulting mixture is agitated with a propeller for a period of about 30 minutes to about 2 hours and after confirming that the wax has become solubilized, the oil-soluble drug is added to the solution, which is then ice-cooled to make a finely dispersed, oil-soluble drug containing wax composition. In this process, as mentioned earlier, one or more substances selected from between POE alkyl ethers and POE-POP alkyl ethers may be used in combination with POE glyceryl ether fatty acid esters as nonionic surfactants, whereupon the rate of wax solubilization can be remarkably increased to achieve an improvement in production efficiency. As a result, the finely divided wax is dispersed in the aqueous dispersion medium, with the oil-soluble drug being contained in the wax.

Needless to say, the process for producing the finely dispersed, oil-soluble drug containing wax composition is in no way limited to the conditions described in the foregoing specific example.

In addition to the production process described above, there is another method that may be employed to prepare the system containing the above-described wax, nonionic surfactant, aqueous dispersion medium, and oil-soluble drug and this method can be performed by using a high-shear emulsifying machine. In the case of using an emulsifying machine capable of imparting a strong shear force, for example, a high-pressure homogenizer, emulsification is preferably performed under a pressure of at least 400 atm but more preferably, it is performed at a temperature not lower than the melting point of the wax under a pressure of at least 600 atm.

[Skin external preparation]

The skin external preparation of the present invention contains the finely dispersed, oil-soluble drug containing wax composition described above. Besides the finely dispersed, oil-soluble drug containing wax composition, ingredients that may be commonly added to skin external preparations and other cosmetics may be added, as appropriate for the need, to the skin external preparation of the present invention. Such optional additive ingredients include but are not limited to humectants such as polyhydric alcohols (e.g., glycerin), perfumes, pH modifiers, antiseptics, various kinds of powders, water-soluble polymers (e.g., natural water-soluble polymers, semisynthetic water-soluble polymers, synthetic water-soluble polymers, and inorganic water-soluble polymers), and silicone-based compounds.

The skin external preparation of the present invention can advantageously be used as various products that are specifically exemplified by lotions, beauty essences in lotion form, beauty essences in jelly form, and skin-care creams. Advantageous dosage forms may be exemplified by but are not limited to clear to semiclear, finely dispersed lotions and thickened jelly products.

EXAMPLES

The present invention is hereunder described in greater detail by means of the following Examples which are by no means intended to limit the invention. Unless otherwise noted, the amounts in which various components are incorporated are indicated by mass% relative to the system to which they are added.

[Example 1: Optimum HLB for the nonionic surfactant]

In the basic formula 1 shown below, the HLB of nonionic surfactants was varied as shown in Table 1 and the state of emulsification of the resulting systems was examined to determine optimum HLB values for the nonionic surfactants used.

| <Basic formula 1> | |
|---|---|
| Carnauba wax | 10 mass% |
| Retinol | 0.1 mass% |
| Nonionic surfactant (See Table 1) | 13.5 mass% |
| Ion-exchange water | bal. |
| Total | 100 mass% |

(Test method)

Finely dispersed, oil-soluble drug containing wax compositions were prepared according to the recipes shown in Table 1 below. To be more specific, the surfactant was dissolved in ion-exchange water and to the solution being heated to 85-95˚C, carnauba was added and the resulting mixture was agitated with a propeller for about 2 hours. Retinol was then added and the mixture was agitated with a propeller for an additional period of about 10 minutes. Thereafter, the mixture was ice-cooled to prepare the composition.

The prepared compositions were allowed to stand at room temperature for an hour and visually observed for their appearance (state of emulsification). The results are also shown in Table 1.

[Table 1]

| Nonionic surfactant in <Basic formula 1> | Amount (mass%) | HLB | State of emulsification |
|---|---|---|---|
| POE(10)behenyl ether | 13.5 | 9 | Phase separation occurred. |
| POE(10)behenyl ether<br>POE(50)lauryl ether | 12.49<br>1.01 | 10 | Creamy |
| POE(10)behenyl ether<br>POE(50)lauryl ether | 11.14<br>2.36 | 11 | Semiclear one-liquid phase |
| POE(15)behenyl ether | 13.5 | 12 | Clear one-liquid phase |
| POE(20)behenyl ether | 13.5 | 13 | Clear one-liquid phase |
| POE(20)behenyl ether<br>POE(30)lauryl ether | 6.75<br>6.75 | 14 | Semiclear one-liquid phase |
| POE(30)behenyl ether | 13.5 | 15 | Creamy |

As is clear from the data in Table 1, uniform emulsion systems could be formed with nonionic surfactants having HLB values of at least 10 but not more than 15. It was also confirmed that clear to semiclear systems of one-liquid phase were obtained with nonionic surfactants having HLB values of 11 to 14 and that, in particular, HLB values of 12 to 13 were required to obtain clear systems.

[Example 2: Types of nonionic surfactants and the state of dispersion of the resulting systems]

In the above-mentioned basic formula 1, POE straight-chain alkyl ethers or POE branched-chain alkyl ethers were used as nonionic surfactants and their HLB values were varied between 9 and 15 by changing the number of moles of adducts in POE; the state of dispersion of the respective systems was evaluated by the criterion defined below. The results are shown in Table 2.

(Test method)

The surfactant was dissolved in ion-exchange water and to the solution being heated to 85-95˚C, carnauba was added and the resulting mixture was agitated with a propeller for about 2 hours. Retinol was then added and the mixture was agitated with a propeller for an additional period of about 10 minutes. Thereafter, the mixture was ice-cooled to prepare a finely dispersed, oil-soluble drug containing wax composition.

The prepared compositions were allowed to stand at room temperature for an hour and visually observed for their appearance. The results are also shown in Table 2.

(Evaluation)

○: A clear one-liquid phase formed.

△: A semiclear or uniform creamy appearance was seen.

×: Phase separation occurred.

[Table 2]

| Nonionic surfactant in <Basic formula 1> | HLB value | | | | | | |
|---|---|---|---|---|---|---|---|
| | 9 | 10 | 11 | 12 | 13 | 14 | 15 |
| POE straight-chain alkyl ether | | | | | | | |

(continued)

| Nonionic surfactant in <Basic formula 1> | HLB value | | | | | | |
|---|---|---|---|---|---|---|---|
| | 9 | 10 | 11 | 12 | 13 | 14 | 15 |
| C12 (lauryl) | ×<br>n=5 | △<br>n=7 | | | ○<br>n=15 | | |
| C16 (cetyl) | ×<br>n=7 | | △<br>n=9 | | | | |
| C18 (stearyl) | ×<br>n=8 | △<br>n=10 | | ○<br>n=15 | | △<br>n=20 | |
| C18 (oleyl) | ×<br>n=8 | △<br>n=10 | | ○<br>n=15 | | | |
| C20 (aralkyl) | ×<br>n=10 | | | | ○<br>n=18 | | |
| C22 (behenyl) | ×<br>n=10 | | | ○<br>n=15 | ○<br>n=20 | | △<br>n=30 |
| POE branched-chain alkyl ether | | | | | | | |
| C18 (isostearyl) | | △<br>n=10 | | ○<br>n=15 | ○<br>n=20 | | |
| C20 (octyldodecyl) | | △<br>n=10 | | ○<br>n=16 | ○<br>n=20 | | |
| C24 (decyltetradecyl) | ×<br>n=10 | | △<br>n=15 | ○<br>n=20 | | △<br>n=15 | |
| POE(20)glyceryl ether isostearic acid ester | | | | | ○<br>n=20 | | ○<br>n=60 |
| Combination of POE(20)glyceryl ether isostearic acid ester and POE (20) straight-chain behenyl alkyl ether | | | | | ○<br>n=20 | | |
| (n: Number of moles of adducts in POE) | | | | | | | |

As is clear from the data in Table 2, when the respective nonionic surfactants were used independently, clear systems of one-liquid phase could be formed by adjusting their HLB values to lie at approximately 12-13. It was also confirmed that a plurality of nonionic surfactants could be used in combination.

[Example 3: Stability with time]

In the basic formula 2 shown below, the nonionic surfactant was varied as shown in Table 3 below and the stability of the resulting systems was evaluated by the criterion defined below.

| <Basic formula 2> | |
|---|---|
| Carnauba wax | 10 mass% |
| Retinol | (For its amount, see Table 3) |
| Nonionic surfactant (See under Table 3) | 15 mass% |
| Ion-exchange water | bal. |
| Total | 100 mass% |

(Test method)

The surfactant was dissolved in ion-exchange water and to the solution being heated to 85-95°C carnauba and retinol were added and the resulting mixture was agitated with a propeller for about 2 hours. Thereafter, the mixture was

ice-cooled to prepare finely dispersed, oil-soluble drug containing wax compositions which were clear and of one-liquid phase. The compositions (samples 1 to 8) were allowed to stand at 50˚C for a week, visually observed for their state, and had their stability with time evaluated by the criterion defined below. The results are also shown in Table 3.

(Evaluation)

○: No change from the state of the as-prepared sample.
×: Phase separation occurred.

[Table 3]

| | Sample | | | | | | | |
|---|---|---|---|---|---|---|---|---|
| | **1** | **2** | **3** | **4** | **5** | **6** | **7** | **8** |
| Carnauba wax | 10 | 10 | 10 | 10 | 10 | 10 | 10 | 10 |
| POE(10)behenyl ether | 5 | - | - | - | - | - | - | 12 |
| POE(15)behenyl ether | - | 12 | 10 | - | - | 5 | - | - |
| POE(20)behenyl ether | - | 12 | - | 5 | 10 | 7 | 9 | 9 |
| POE(30)behenyl ether | - | - | - | - | - | 7 | - | - |
| POE(40) hydrogenated castor oil | 10 | - | - | 10 | 3.5 | - | - | - |
| POE(20)glyceryl ether isostearic acid ester | - | - | - | - | - | - | - | 6.5 |
| POE(4.5)lauryl acetate ether(ca. 20 mass% effective content) | - | - | 1.5 | - | - | 7.5 | - | - |
| Stearoylmethyl taurate | - | - | - | 0.1 | 0.5 | - | - | - |
| Retinol | 0.1 | 0.1 | 0.1 | 0.1 | 0.1 | 0.1 | 1.0 | 0.1 |
| Ion-exchange water | bal. | bal. | bal. | bal. | bal. | bal. | bal. | bal. |
| Stability | ○ | ○ | ○ | ○ | ○ | ○ | × | ○ |

As is clear from the data shown in Table 3, sample 7 in which the mass ratio of the nonionic surfactant to the wax was less than unity experienced phase separation and failed to exhibit good stability.

[Example 4: Effect of controlling the release of oil-soluble drug (tocopherol acetate)]

Finely dispersed, oil-soluble drug containing wax compositions (samples X to Z) were prepared according to the formulas shown in Table 4 below and then evaluated for the amount in which tocopherol acetate permeated through the skin. To evaluate the amount in which tocopherol acetate would permeate through the skin, each sample was applied to the skin and a measurement was performed on the as-prepared sample and then repeated every 5 hr until 20 hours had passed after application. The results are shown in Fig. 1. Note that samples X to Z each had good stability.
[Table 4]

| | Sample | | |
|---|---|---|---|
| | **X** | **Y** | **Z** |
| Carnauba wax | - | 10 | 3 |
| Carnauba wax | - | - | 7 |
| Pentaerythrityl tetraethylhexanoate | 10 | - | - |

(continued)

| | Sample | | |
|---|---|---|---|
| | **X** | **Y** | **Z** |
| Behenyl alcohol | 3.3 | - | - |
| Stearyl alcohol | 0.9 | - | - |
| POE(10)behenyl ether | - | - | 5 |
| POE(20)behenyl ether | 10 | 10 | 10 |
| POE(20)glyceryl ether isostearic acid ester | - | 5 | - |
| Tocopherol acetate | 0.1 | 0.1 | 0.1 |
| Ion-exchange water | bal. | bal. | bal. |

As Fig. 1 shows, samples Y and Z of finely dispersed wax were capable of effectively controlling the amount of initial permeation of tocopherol acetate as compared with sample X, or the conventional O/W emulsion in which the oil phase assumed a liquid state at ordinary temperatures, and consequently, the amount of skin permeation of tocopherol acetate was held virtually constant.

[Examples 5 to 8 and Comparative Examples 1 and 2: Evaluation of skin irritation and use properties]

Samples were prepared according to the recipes shown in Table 5 below (Examples 5-8 and Comparative Examples 1 and 2) and applied to the skin of 20 expert panelists who were asked to evaluate the samples for their skin irritating effect and feeling during use according to the criterion defined below. The results are also shown in Table 5. All the samples of Examples 5-8 and Comparative Examples 1 and 2 had good stability.

(Evaluation of skin irritating effect)

○: At least 15 out of the 20 panelists answered "not irritating to the skin".

△: Ten to 14 out of the 20 panelists answered "not irritating to the skin".

×: Not more than 9 out of the 20 panelists answered "not irritating to the skin".

(Freshness)

○: At least 15 out of the 20 panelists answered "fresh".
△: Ten to 14 out of the 20 panelists answered "fresh".
×: Not more than 9 out of the 20 panelists answered "fresh".

[Table 5]

| | Example | | | | Comparative Example | |
|---|---|---|---|---|---|---|
| | **5** | **6** | **7** | **8** | **1** | **2** |
| Carnauba wax | 2 | 2 | 10 | 1.5 | - | - |
| Pentaerythrityl tetraethylhexanoate | - | - | - | - | 10 | 10 |
| Behenyl alcohol | - | - | - | - | 3.3 | 3.3 |
| Stearyl alcohol | - | - | - | - | 0.9 | 0.9 |

(continued)

| | Example | | | | Comparative Example | |
|---|---|---|---|---|---|---|
| | **5** | **6** | **7** | **8** | **1** | **2** |
| POE(20)behenyl ether | - | 2.3 | 11.5 | 1.8 | 10 | 30 |
| POEglyceryl ether isostearic acid ester | 2.3 | - | - | 1.0 | - | - |
| Dipropylene glycol | - | - | - | 5.0 | - | - |
| 1,3-Butylene glycol | - | - | - | 7.0 | - | - |
| Retinol | 0.1 | 0.1 | 0.1 | 0.1 | 0.1 | 0.1 |
| Ion-exchange water | bal. | bal. | bal. | bal. | bal. | bal. |
| Skin irritating effect | ○ | ○ | ○ | ○ | × | ○ |
| Freshness | ○ | ○ | ○ | ○ | △ | × |

[Example 9 and Comparative Example 3: Solubilizing rate of wax]

According to Table 6 shown below, a surfactant or surfactants and dipropylene glycol were dissolved in ion-exchange water, and to the solution being heated to 85-95˚C, carnauba wax added and the resulting mixture was agitated with a propeller and the time it took for the carnauba wax to be solubilized was measured. The results are also shown in Table 6.

[Table 6]

| | Example 9 | Comparative Example 3 |
|---|---|---|
| Carnauba wax | 10 | 10 |
| POE(20)behenyl ether | 12 | 18 |
| POE(20)glyceryl ether isostearic acid ester | 6 | - |
| Dipropylene glycol | 6 | 6 |
| Ion-exchange water | bal. | bal. |
| Time to wax solubilization (min) | 30 | 60 |

As is clear from the data in Table 6, the time it took for the wax to become solubilized in Example 9 where POE (20)behenyl ether and POE(20)glyceryl ether isostearic acid ester were used in combination as nonionic surfactants could be made considerably shorter than in Comparative Example 3 which used only one kind of nonionic surfactant, i.e., POE(20)behenyl ether.

[Examples 10 and 11: Percent residue of oil-soluble drug (retinol)]

Samples were prepared according to the recipes shown in Table 7 below and stored at 50 ˚C for 2 months; thereafter, the percent residue of the oil-soluble drug (retinol) was measured by the following method. The results are also shown in Table 7.

(Measurement of percent residue of retinol)

Half a gram was precisely weighed from each of the samples of Examples 10 and 11; 5 mL of xylene was added to each aliquot and the mixture was heated on a water bath to achieve dispersion; 30 mL of dibutylhydroxytoluene in acetone solution (0.5%) was added and the mixture was further heated to about 80˚C and then left to cool to ordinary temperatures; an internal standard solution of stearyl glycyrrhizinate (= stearyl glycyrrhizinate that was precisely weighed in 2 g and dissolved in acetone to make a solution that accurately weighed 3000 mL) was accurately weighed to 5 mL, added to the cooled mass, and mixing was done. A 2-μL portion of the liquid mixture was sampled as a test solution; the ratio of the peak area of retinol to that of the internal standard was determined and the mass ratio was determined

from a preliminarily prepared calibration curve; the obtained values were substituted into the following equation 2 to determine the amount of retinol (IU/g).

[Equation 2]

Amount of retinol (IU/g) = Mass ratio determined from the calibration curve times the amount of stearyl glycyrrhizinate (mg) in 5 mL of the internal standard solution times the international unit of analytical retinol (IU/g), divided by the amount of a collected sample (g) and multiplied by 1000.

<Test conditions>

Detector: UV spectrophotometer (measurement wavelength: 280 nm for 0-10 min, and 254 nm thereafter).
Column: Stainless steel tube having an inside diameter of 3.0 mm and a length of 50 mm was packed with 3-μm silica gel particles provided with a silicone resin coat to which octadecyl groups were later bonded chemically.
Column temperature: Constant temperature near 40˚C.
Mobile phase A: Water
Mobile phase B: Methanol

Feeding of the mobile phases: Density gradient control was performed by changing the mixing ratio of two mobile phases A and B as follows:

0 to 5 min after injection: mobile phase A (15→0); mobile phase B (85→100)
After the passage of 5 min onward: mobile phase A (0); mobile phase B (100)
The flow rate took two constant values; for the period of 0 to 5 minutes after injection, it was near 0.4 mL/min and after the passage of 5 minutes, it was near 0.6 mL.

<Constructing a calibration curve>

A tenth of a gram was precisely weighed from analytical retinol and dissolved in dibutylhydroxytoluene in acetone solution (1→200) to make a solution that accurately weighed 200 mL. The solution was sampled in accurate amounts of 0.1 mL, 0.25 mL, 0.5 mL, and 1 mL; to each aliquot, 5 mL of internal standard solution of stearyl glycyrrhizinate was accurately added and mixed with dibutylhydroxytoluene in acetone solution (1→200) to make 40 mL of a standard solution for constructing a calibration curve. A 5-μL portion of the standard solution for constructing a calibration curve was sampled and tested under the conditions described above; by plotting the peak area ratio (retinol/internal standard) for the resulting chromatogram on the horizontal axis and the mass ratio (retinol/internal standard) on the vertical axis, a calibration curve was constructed.

[Table 7]

| | Example 10 | Example 11 |
|---|---|---|
| Dipropylene glycol | 5 | 5 |
| 1,3-Butylene glycol | 7 | 7 |
| POE(20)behenyl ether | 1.8 | 1.8 |
| POE(20)glyceryl ether isostearic acid ester | 1 | 1 |
| Carnauba wax | 1.5 | 1.5 |
| Retinol | 0.01 | 0.1 |
| Citric acid | 0.01 | 0.01 |
| Sodium citrate | 0.09 | 0.09 |
| EDTA • 3Na • $2H_2O$ | 0.02 | 0.02 |
| Tocopherol | 0.05 | 0.05 |

(continued)

| | Example 10 | Example 11 |
|---|---|---|
| Dibutylhydroxytoluene | 0.05 | 0.05 |
| Phenoxyethanol | 0.5 | 0.5 |
| Ion-exchange water | bal. | bal. |
| Residual retinol (%) after storage at 50˚C for 2 months | 88 | 91 |

As is clear from the data in Table 7, the use of POE(20)behenyl ether and POE(20)glyceryl ether isostearic acid ester in combination as nonionic surfactants led to a satisfactory percent residue of the oil-soluble drug (retinol) even after the storage at 50 ˚C for 2 months whether it was contained in an amount of 0.01 mass% or 0.1 mass%.
Set forth below are formulae that can be applied in the present invention.

[Compounding formula 1: Lotion]

| (Ingredients to be compounded) | (Mass%) |
|---|---|
| (1) Carnauba wax | 1.5 |
| (2) POE(20)behenyl ether | 1.8 |
| (3) Retinol | 0.1 |
| (4) Dibutylhydroxytoluene | 0.05 |
| (5) Tocopherol acetate | 0.05 |
| (6) Glycerin | 5 |
| (7) Ion-exchange water | bal. |
| (8) Perfume | q.s. |
| (9) Phenoxyethanol | 0.5 |

(Method of production)

Ingredients (1) to (5) and(8), as well as portions of ingredients (6) and (7) were mixed under stirring at about 95 ˚C until the mixture became clear; thereafter, it was ice-cooled to make a fine dispersion of the wax; the fine dispersion of the wax was added to a mixture of the remaining portions of (6) and (7) with ingredient (9) to make a lotion.

[Compounding formula 2: Lotion]

| (Ingredients to be compounded) | (Mass%) |
|---|---|
| (1) Carnauba wax | 1.5 |
| (2) POE(10)behenyl ether | 0.9 |
| (3) POE(30)behenyl ether | 0.9 |
| (4) POE(30)glyceryl ether isostearic acid ester | 0.05 |
| (5) Candelilla wax | 0.5 |
| (6) Ion-exchange water | bal. |
| (7) Retinol acetate | 0.05 |
| (8) Isobutyl resorcine | 0.3 |
| (9) Vitamin C dipalmitate | 0.05 |
| (10) Paraben | 0.3 |
| (11) Dipropylene glycol | 5 |

(Method of production)

Ingredient (1) as well as portions of ingredients (2)-(4), (6) and (11) were mixed under stirring at about 95 ˚C until the mixture became clear; thereafter, portions of ingredients (7)-(9) were added and the mixture was ice-cooled to make drug composition A which was a fine dispersion of the wax. In a separate step, ingredient (5), the remaining portions of (2)-(4), and portions of (6) and (11) were mixed under stirring at 95 ˚C until the mixture became clear; then, the remaining portions of (7)-(9) were added and the mixture was ice-cooled to make drug composition B which was also a fine dispersion of the wax. The remaining portions of (6) and (11) were mixed with ingredient (10), then with drug compositions A and B to make a lotion having different wax contents in A and B.

[Compounding formula 3: Anti-aging lotion]

| (Ingredients to be compounded) | (Mass%) |
|---|---|
| (1) Carnauba wax | 1.5 |
| (2) POE(10)behenyl ether | 0.1 |
| (3) POE(20)behenyl ether | 2 |
| (4) POE(30)behenyl ether | 0.1 |
| (5) POE(1)POE(15)behenyl ether | 0.2 |
| (6) POE(20)glyceryl ether isostearic acid ester | 0.2 |
| (7) POE(60) hydrogenated castor oil | 0.2 |
| (8) Retinol | 0.01 |
| (9) Retinol acetate | 0.01 |
| (10) Retinol palmitate | 0.01 |
| (11) α-Tocopherol | 0.01 |
| (12) Tocopherol acetate | 0.05 |
| (13) Dibutylhydroxytoluene | 0.02 |
| (14) Sodium ascorbate | 0.1 |
| (15) Ascorbic acid phosphate sodium salt | 0.01 |
| (16) Ascorbic acid 2-glucoside | 0.5 |
| (17) Tranexamic acid | 2 |
| (18) Erythritol | 0.01 |
| (19) Xylitol | 0.01 |
| (20) Glycerin | 5 |
| (21) Dipropylene glycol | 5 |
| (22) POE-POP dimethyl ether | 0.5 |
| (23) Phenoxyethanol | 0.5 |
| (24) Trisodium edentate | 0.02 |
| (25) Citric acid | q.s. |
| (26) Sodium citrate | q.s. |
| (27) Potassium hydroxide | q.s. |
| (28) Methyl polysiloxane | 2 |
| (29) Behenyl alcohol | 0.1 |
| (30) Sodium stearoylmethyl taurate | 0.05 |
| (31) Ethanol | 5 |
| (32) Perfume | q.s. |
| (33) Ion-exchange water | bal. |

[Compounding formula 4: Skin-lightening jelly]

| (Ingredients to be compounded) | (Mass%) |
|---|---|
| (1) Carnauba wax | 0.5 |
| (2) Candelilla wax | 0.3 |
| (3) Rice wax | 0.2 |
| (4) Beeswax | 0.1 |
| (5) Microcrystalline wax | 0.1 |
| (6) Paraffin wax | 0.1 |
| (7) Petrolatum | 0.1 |
| (8) Behenyl alcohol | 0.1 |
| (9) Stearyl alcohol | 0.03 |
| (10) POE(10)behenyl ether | 0.1 |
| (11) POE(20)behenyl ether | 1.5 |
| (12) POE(30)behenyl ether | 0.1 |
| (13) POE(20)glyceryl ether isostearic acid ester | 0.1 |
| (14) Retinol palmitate | 0.01 |
| (15) $\alpha$-Tocopherol | 0.01 |
| (16) Tetraisopalmitoylascorbyl (= oil-soluble vitamin C) | 0.01 |
| (17) Isobutyl resorcine | 0.01 |
| (18) Linoleic acid | 0.03 |
| (19) Linolenic acid | 0.02 |
| (20) Ascorbic acid phosphate sodium salt | 0.01 |
| (21) Ascorbic acid phosphate magnesium salt | 0.01 |
| (22) Ascorbic acid 2-glucoside | 2 |
| (23) Ethyl ascorbate | 0.01 |
| (24) Pantothenylethyl ether | 0.01 |
| (25) Arbutin | 0.01 |
| (26) Methyl tranexamate amide salt | 1 |
| (27) Dipotassium glycyrrhizinate | 0.01 |
| (28) 4-Methoxysalicylic acid salt | 1 |
| (29) Glycerin | 1 |
| (30) Dipropylene glycol | 10 |
| (31) Polyoxyethylene glycol | 1 |
| (32) Paraoxybenzoic acid ester | 0.15 |
| (33) Phenoxyethanol | 0.3 |
| (34) Trisodium edentate | 0.02 |
| (35) Citric acid | q.s. |
| (36) Sodium citrate | q.s. |
| (37) Potassium hydroxide | q.s. |
| (38) Methyl polysiloxane | 2 |
| (39) Phenyl polysiloxane | 1 |
| (40) Sodium stearoylmethyl taurate | 0.05 |
| (41) Carboxyvinyl polymer | 2 |
| (42) Acrylic acid/alkyl ($C_{10}$-$_{30}$) acrylate copolymer | 0.5 |
| (43) Hydroxyethyl cellulose | 0.1 |
| (44) Methyl cellulose | 1 |
| (45) 2-Methacryloyloxyethylphosphorylcholine/ butyl methacrylate copolymer | 0.1 |
| (46) Ethanol | 10 |
| (47) Perfume | q.s. |
| (48) Ion-exchange water | bal. |

[Compounding formula 5: Skin roughness ameliorating cream]

| (Ingredients to be compounded) | (Mass%) |
|---|---|
| (1) Carnauba wax | 0.8 |
| (2) Candelilla wax | 0.2 |
| (3) Beeswax | 0.1 |
| (4) Microcrystalline wax | 0.1 |
| (5) Petrolatum | 0.1 |
| (6) Liquid paraffin | 0.1 |
| (7) Squalane | 0.1 |
| (8) POE(20)behenyl ether | 2 |
| (9) POP(1)POE(15)behenyl ether | 0.2 |
| (10) POE(20)glyceryl ether isostearic acid ester | 0.8 |
| (11) α-Tocopherol | 0.01 |
| (12) Tocopherol acetate | 0.01 |
| (13) Stearyl glycyrrhetinate | 0.01 |
| (14) Oil-soluble vitamin B | 0.01 |
| (15) Dibutylhydroxytoluene | 0.01 |
| (16) Benzyl nicotinate | 0.01 |
| (17) Tranexamic acid | 0.5 |
| (18) Methyl tranexamate amide salt | 0.1 |
| (19) 4-Methoxysalicylic acid salt | 0.5 |
| (20) Nicotinic acid | 0.01 |
| (21) Nicotinic acid amide | 0.01 |
| (22) Hydroxyproline | 0.01 |
| (23) Serine | 0.01 |
| (24) Thiotaurine | 0.01 |
| (25) Arginine | 0.01 |
| (26) Trimethylglycine | 0.01 |
| (27) Erythritol | 5 |
| (28) Glycerin | 2 |
| (29) Butylene glycol | 5 |
| (30) Dipropylene glycol | 5 |
| (31) Polyoxyethylene glycol | 2 |
| (32) Phenoxyethanol | 0.5 |
| (33) Trisodium edentate | 0.02 |
| (34) Citric acid | q.s. |
| (35) Sodium citrate | q.s. |
| (36) Potassium hydroxide | q.s. |
| (37) Methyl polysiloxane | 2 |
| (38) Phenyl polysiloxane | 1 |
| (39) Decamethylcyclopentasiloxane | 5 |
| (40) Cetyl 2-ethylhexanoate | 1 |
| (41) Bleached beeswax | 1 |
| (42) Batyl alcohol | 1.65 |
| (43) Behenyl alcohol | 0.77 |
| (44) Sodium stearoylmethyl taurate | 0.01 |
| (45) Polyvinyl alcohol | 0.05 |
| (46) Ethanol | 5 |
| (47) Perfume | q.s. |
| (48) Ion-exchange water | bal. |

[Compounding formula 6: Moisturizing jelly]

| (Ingredients to be compounded) | (Mass%) |
|---|---|
| (1) Carnauba wax | 0.5 |
| (2) Rice wax | 0.4 |
| (3) Paraffin wax | 0.1 |
| (4) POE cholesterol ether | 0.1 |
| (5) Glyceryl stearate | 0.05 |
| (6) Hydrogenated castor oil | 0.1 |
| (7) Behenyl alcohol | 0.2 |
| (8) Batyl alcohol | 0.05 |
| (9) Stearic acid | 0.05 |
| (10) Triglycerin | 0.05 |
| (11) Glycerol Trioctanoate | 0.05 |
| (12) Paraffin | 0.1 |
| (13) POE(10)behenyl ether | 0.7 |
| (14) POE(30)behenyl ether | 0.8 |
| (15) POP(1)POE(15)behenyl ether | 0.2 |
| (16) POE(20)glyceryl ether isostearic acid ester | 0.01 |
| (17) POE(60) hydrogenated castor oil | 0.5 |
| (18) Tocopherol acetate | 0.01 |
| (19) Stearyl glycyrrhetinate | 0.01 |
| (20) Ubiquinone | 0.01 |
| (21) β-Carotene | 0.01 |
| (22) Ergocalcipherol (= vitamin $D_2$) | 0.005 |
| (23) Cholecalcipherol (= vitamin $D_3$) | 0.005 |
| (24) γ-Orizanol | 0.01 |
| (25) Ascorbic acid phosphate magnesium salt | 1 |
| (26) Nicotinic acid | 0.01 |
| (27) Urea | 2 |
| (28) Hyaluronic acid | 0.001 |
| (29) Acetylated hyaluronic acid | 0.001 |
| (30) Trehalose | 1 |
| (31) Erythritol | 1 |
| (32) Xylitol | 1 |
| (33) Glycerin | 5 |
| (34) Polyoxyethylene glycol | 5 |
| (35) POE-POP dimethyl ether | 1 |
| (36) Phenoxyethanol | 0.5 |
| (37) Trisodium edentate | 0.02 |
| (38) Citric acid | q.s. |
| (39) Sodium citrate | q.s. |
| (40) Potassium hydroxide | q.s. |
| (41) Methyl polysiloxane | 1 |
| (42) Phenyl polysiloxane | 1 |
| (43) Behenyl alcohol | 0.1 |
| (44) Sodium stearoylmethyl taurate | 0.05 |
| (45) Agar | 2.5 |
| (46) Dimethyl acrylamide/sodium acryloyldimethyl taurate crosspolymer | 5 |
| (47) Perfume | q.s. |
| (48) Ion-exchange water | bal. |

[Compounding formula 7: UV preventing jelly]

| (Ingredients to be compounded) | (Mass%) |
|---|---|
| (1) Carnauba wax | 0.4 |
| (2) Beeswax 0.3 | 0.5 |
| (3) Paraffin wax | 0.1 |
| (4) POE cholesterol ether | 0.1 |
| (5) Hydrogenated castor oil | 0.1 |
| (6) Behenyl alcohol | 0.5 |
| (7) Batyl alcohol | 0.1 |
| (8) Stearic acid | 0.05 |
| (9) Triglycerin | 0.05 |
| (10) Pentaerythritol tetraoctanoate | 0.1 |
| (11) POE(20)behenyl ether | 1.8 |
| (12) POE(20)glyceryl ether isostearic acid ester | 1.5 |
| (13) POE(60)glyceryl ether isostearic acid ester | 0.03 |
| (14) Tocopherol acetate | 0.01 |
| (15) Stearyl glycyrrhetinate | 0.01 |
| (16) Isobutyl resorcine | 0.01 |
| (17) Octocrylene | 0.05 |
| (18) Octyl methoxycinnamate | 0.05 |
| (19) Tranexamic acid | 1 |
| (20) Methyl tranexamate amide salt | 1 |
| (21) Nicotinic acid amide | 0.01 |
| (22) Hydroxyproline | 0.01 |
| (23) Glycerin | 10 |
| (24) Butylene glycol | 5 |
| (25) Polyoxyethylene glycol | 1 |
| (26) POE-POP dimethyl ether | 5 |
| (27) Paraoxybenzoic acid ester | 0.1 |
| (28) Phenoxyethanol | 0.5 |
| (29) Trisodium edentate | 0.02 |
| (30) Citric acid | q.s. |
| (31) Sodium citrate | q.s. |
| (32) Potassium hydroxide | q.s. |
| (33) Methyl polysiloxane | 3 |
| (34) Phenyl polysiloxane | 2 |
| (35) Carboxyvinyl polymer | 5 |
| (36) Acrylic acid/alkyl ($C_{10-30}$) acrylate copolymer | 0.5 |
| (37) KELTROL® | 0.05 |
| (38) Ethanol | 5 |
| (39) Perfume | q.s. |
| (40) Ion-exchange water | bal. |

BRIEF DESCRIPTION OF THE DRAWING

[Fig. 1]
A graph showing that the amount of permeation of an oil-soluble drug could be effectively controlled in Example 4.

INDUSTRIAL APPLICABIIITY

The skin external preparations of the present invention are **characterized in that** the initial permeation rate of oil-soluble drugs is controlled to achieve their sustained release to thereby reduce skin irritation and that they also possess a fresh feel while featuring high degrees of safety and stability.

## Claims

1. A skin external preparation which comprises a finely dispersed, oil-soluble drug containing wax composition, the composition containing a solid or semisolid wax, a nonionic surfactant, an aqueous dispersion medium, and an oil-soluble drug, the mass ratio of the nonionic surfactant to the wax being 1.0 or more, and the wax, with the oil-soluble drug contained therein, being finely dispersed in solid or semisolid form in the aqueous dispersion medium.

2. The skin external preparation according to claim 1, wherein the oil-soluble drug is one or more substances selected from among vitamin A and its derivatives, vitamin $B_2$ derivatives, vitamin $B_6$ derivatives, vitamin D and its derivatives, vitamin E and its derivatives, essential fatty acids, ubiquinone and its derivatives, K vitamins, resorcin derivatives, glycyrrhetinic acid and its derivatives, oil-soluble vitamin C derivatives, steroid compounds, benzyl nicotinate, trichlorocarbanilide, trichlorohydroxydiphenylether, stearyl glycyrrhetinate, y-orizanol, and dibutylhydroxytoluene.

3. The skin external preparation according to claim 1, wherein the weight-averaged HLB for all nonionic surfactants in the finely dispersed, oil-soluble drug containing wax composition is in the range of 10-15.

4. The skin external preparation according to claim 1, wherein one or more substances selected from among polyoxyethylene alkyl ethers, polyoxyethylene-polyoxypropylene alkyl ethers, polyoxyethylene glyceryl ether fatty acid esters, and polyoxyethylene castor oil or polyoxyethylene hydrogenated castor oil and their derivatives are contained as the nonionic surfactant.

5. The skin external preparation according to claim 1, wherein polyoxyethylene alkyl ethers and/or polyoxyethylene-polyoxypropylene alkyl ethers and polyoxyethylene glyceryl ether fatty acid esters are contained as nonionic surfactants.

6. The skin external preparation according to claim 4 or 5, wherein the polyoxyethylene alkyl ethers and polyoxyethylene-polyoxypropylene alkyl ethers are one or more substances selected from among compounds represented by the following formulas (I) and/or (II):

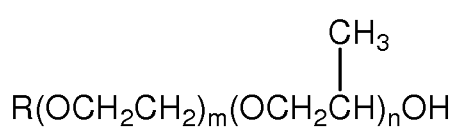

(I)

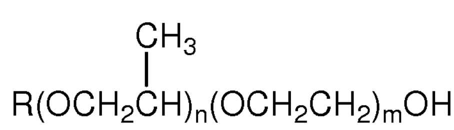

(II)

[in the formulas (I) and (II), R represents an alkyl or alkenyl group having 12-24 carbon atoms, m represents a number of 5-30, and n represents a number of 0-5].

7. The skin external preparation according to claim 1, wherein the finely dispersed, oil-soluble drug containing wax composition is obtained by such a process that a system containing a wax that is solid or semisolid at ordinary temperatures, a nonionic surfactant, an aqueous dispersion medium, and an oil-soluble drug, with the mass ratio

of the nonionic surfactant to the wax being 1.0 or more, is heated to a temperature not lower than the melting point of the wax but within the solubilizing temperature range so that the wax becomes solubilized and the system is thereafter cooled to ordinary temperatures.

[FIG. 1]

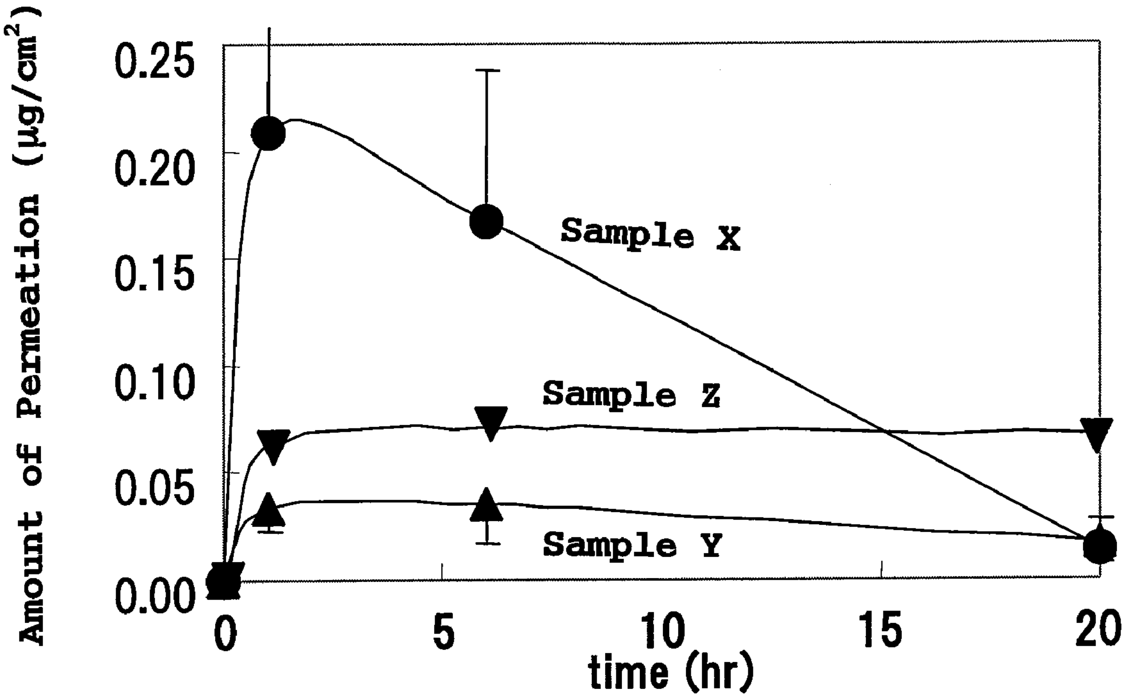
Amount of Permeation (μg/cm²)
0.25
0.20
0.15
0.10
0.05
0.00
0
5
10
15
20
time (hr)
Sample X
Sample Z
Sample Y

**INTERNATIONAL SEARCH REPORT**

International application No.
PCT/JP2008/072201

A. CLASSIFICATION OF SUBJECT MATTER
*A61K47/44*(2006.01)i, *A61K8/04*(2006.01)i, *A61K8/92*(2006.01)i, *A61K31/07*(2006.01)i, *A61K31/355*(2006.01)i, *A61K31/375*(2006.01)i, *A61K45/00*(2006.01)i, *A61K47/34*(2006.01)i, *A61P17/00*(2006.01)i, *A61Q19/00*(2006.01)i

According to International Patent Classification (IPC) or to both national classification and IPC

B. FIELDS SEARCHED

Minimum documentation searched (classification system followed by classification symbols)
A61K47/44, A61K8/04, A61K8/92, A61K31/07, A61K31/355, A61K31/375, A61K45/00, A61K47/34, A61P17/00, A61Q19/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2006-516029 A (Cognis IP Management GmbH.),<br>15 June, 2006 (15.06.06),<br>Claims 1, 3, 9, 14, 23; Par. Nos. [0001], [0007], [0025], [0045], [0064]<br>& US 2006/0116524 A1 & EP 1581170 A<br>& WO 2004/062630 A1 & DE 10300506 A | 1-7<br>1-7 |
| X<br>Y | JP 2004-523519 A (Cognis Deutschland GmbH & Co., KG. ),<br>05 August, 2004 (05.08.04),<br>Claims 1, 3; Par. Nos. [0006], [0022] to [0029], [0034], [0036], [0047], [0052] to [0053]<br>& US 2004/0086470 A1 & EP 1367981 A<br>& WO 2002/056839 A2 & DE 10102005 A | 1-7<br>1-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 December, 2008 (24.12.08) | 13 January, 2009 (13.01.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2008/072201

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2007-531777 A (Cognis IP Management GmbH.),<br>08 November, 2007 (08.11.07),<br>Claim 8; Par. Nos. [0002], [0023] to [0024],<br>[0043], [0051]<br>& US 2008/0051470 A1 & EP 1732877 A<br>& WO 2005/097056 A & DE 102004017222 A | 1-7<br>1-7 |
| Y | JP 2004-083437 A (Shiseido Co., Ltd.),<br>18 March, 2004 (18.03.04),<br>Claims 1, 4; Par. Nos. [0009], [0014], [0023],<br>[0027] to [0031]<br>(Family: none) | 1-7 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11113487 A
- JP 10324617 A
- JP 11286418 A
- JP 11263915 A
- JP 4230616 A
- JP 3002112 A